(19) Europäisches Patentamt
European Patent Office
Office européen des brevets

(11) **EP 4 541 344 A2**

(12) **DEMANDE DE BREVET EUROPEEN**

(43) Date de publication:
**23.04.2025 Bulletin 2025/17**

(21) Numéro de dépôt: **25163291.5**

(22) Date de dépôt: **01.10.2019**

(51) Classification Internationale des Brevets (IPC):
***A61K 8/19*** *(2006.01)*

(52) Classification Coopérative des Brevets (CPC):
**A61K 8/8176; A61K 8/19; A61Q 17/04**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.10.2018 FR 1859066**

(62) Numéro(s) de document de la (des) demande(s) initiale(s) en application de l'article 76 CBE:
**19795294.8 / 3 860 722**

(71) Demandeur: **Bionuclei**
**13290 Aix-en-Provence (FR)**

(72) Inventeur: **La désignation de l'inventeur n'a pas encore été déposée**

(74) Mandataire: **Cabinet Laurent & Charras**
**Le Contemporain**
**50 Chemin de la Bruyère**
**69574 Dardilly Cedex (FR)**

Remarques:
Cette demande a été déposée le 12-03-2025 comme demande divisionnaire de la demande mentionnée sous le code INID 62.

(54) **BARRIERE PHOTONIQUE A USAGE TOPIQUE COMPRENANT DES COLLOÏDES D'OXYDE DE BISMUTH**

(57) La présente invention concerne une composition topique créant une barrière photonique allant du rayonnement ultraviolet au rayonnement visible comprenant des colloïdes d'oxyde de bismuth $Bi_2O_3$ sous forme cristalline dopés avec un métal.

**Description**

L'invention concerne une barrière photonique allant du rayonnement UV au visible, plus précisément une composition topique contenant des colloïdes d'oxyde de bismuth dopé, notamment l'oxyde de bismuth $\alpha$-$Bi_2O_3$, éventuellement sous forme greffée avec un polymère et leur utilisation.

Le domaine d'utilisation de la présente invention concerne notamment le domaine de la cosmétique et des dispositifs médicaux, et plus particulièrement celui de la protection vis-à-vis du rayonnement électromagnétique.

Il est bien connu que les ultraviolets A (UV-A ; 400-315 nm) représentent 95% des UV qui touchent la surface de la Terre. Ils entraînent le vieillissement prématuré de la peau *via* la production de radicaux libres et donc d'un phénomène de stress oxydatif.

Les ultraviolets B (UV-B ; 315-280 nm) sont plus énergétiques que les UV-A. Néanmoins, ils sont partiellement filtrés par l'atmosphère et représentent 5% des UV reçus sur la Terre. Ils sont responsables des érythèmes actiniques et contribuent également à accélérer le vieillissement de la peau par la production d'un stress oxydatif cellulaire résultant de la génération de radicaux libres.

Les ultraviolets C (UV-C ; 280-100 nm) sont encore plus énergétiques que les UV-B. En revanche, ils sont quasiment intégralement filtrés par la couche d'ozone et ne font pas l'objet d'une attention particulière dans le domaine des filtres solaires.

Les ultraviolets visibles (UV-V ; 400-490 nm) sont moins énergétiques que les autres ultraviolets. Cependant, ce sont les ultraviolets qui pénètrent le plus profondément la peau, allant jusqu'au derme réticulaire et pouvant provoquer des dommages cellulaires importants.

Au sens de l'invention, par « ultraviolets visibles ou UV-V », on désigne le rayonnement visible proche du rayonnement ultraviolet, de préférence de 400 à 490 nm, avantageusement de 400 à 450 nm, encore plus avantageusement de 400 à 420 nm.

L'ensemble des UV est impliqué dans les mécanismes de carcinogénèse.

De manière générale, les compositions de protection solaire comprennent au moins un filtre solaire qui peut être organique ou minéral.

A titre d'exemple et de manière non limitative, les filtres solaires organiques peuvent être des composés appartenant aux familles suivantes : aminobenzoates, cinnamates, salicylates, benzophénones, phényl benzotriazoles...

Les filtres solaires minéraux incluent notamment le dioxyde de titane ($TiO_2$) et l'oxyde de zinc (ZnO).

Ce type de protection permet de limiter les effets néfastes des ultraviolets.

Les différents filtres solaires mentionnés ci-dessus ne permettent pas de couvrir toute la gamme des ultraviolets auxquels les êtres humains peuvent être exposés. En effet, un filtre est généralement actif pour une plage restreinte de longueur d'onde. A titre d'exemple, le dioxyde de titane et l'oxyde de zinc ne filtrent pas tous les UV-A, ou encore un filtre organique de type éthylhexyl triazone ne filtre que les UV-B.

En ce qui concerne plus spécifiquement la protection par filtration à l'égard des UV-V ou ultraviolets visibles, c'est-à-dire le rayonnement visible proche du rayonnement ultraviolet, de préférence de 400 à 490 nm, avantageusement de 400 à 450 nm, encore plus avantageusement de 400 à 420 nm, elle n'a jusqu'à ce jour pas fait l'objet d'un développement spécifique efficace.

Afin de remédier à ce problème, les compositions solaires associent généralement plusieurs filtres solaires et des antioxydants qui neutralisent les effets avals des UV, principalement UV-A (400 à 315 nm) et UV-V (400 à 490 nm). Il peut s'agir d'un mélange de filtres solaires organiques et/ou minéraux.

Cependant, la combinaison de plusieurs filtres avec de nombreux excipients peut engendrer des problèmes d'interactions entre les différents éléments, affectant l'efficacité de la protection. D'autre part, les filtres organiques peuvent être facilement absorbés par la peau. Or, certains sont suspectés d'avoir des effets délétères pour la santé humaine et notamment d'être des perturbateurs endocriniens. Enfin, les différents filtres peuvent subir des dégradations limitant là encore, la protection solaire, voire générant des dommages à l'organisme.

Le problème que se propose de résoudre l'invention est celui de mettre au point une composition visant à protéger la peau du rayonnement ultraviolet et qui ne présente pas les inconvénients présentés ci-dessus.

L'oxyde de bismuth est généralement utilisé dans le domaine de l'imagerie médicale, en tant qu'opacifiant des rayons X, ou dans le domaine de l'énergie, par exemple dans l'électrolyte des piles à combustible.

Le Demandeur a remarqué que, de manière tout à fait inattendue, la forme non amorphe de l'oxyde de bismuth, c'est-à-dire la forme cristalline, peut être utilisée dans une composition cosmétique, notamment en tant que filtre du rayonnement ultraviolet.

Selon un premier aspect, la présente invention concerne une composition topique comprenant des colloïdes d'oxyde de bismuth $Bi_2O_3$ sous forme cristalline dopés, avec un métal.

Cette composition crée une barrière photonique allant du rayonnement ultraviolet au rayonnement visible, de préférence de 200 à 490 nm, avantageusement de 200 à 450 nm, encore plus avantageusement de 200 à 420 nm.

Grâce à la présence des colloïdes d'oxyde de bismuth $Bi_2O_3$ dopé sous forme cristalline, cette composition assure une protection photonique en bloquant une partie du rayonnement électromagnétique significativement plus étendue que les filtres du marché. La présente invention procure donc un avantage indéniable par rapport aux compositions de l'art antérieur qui nécessitent l'utilisation de plusieurs filtres organiques et/ou minéraux pour bloquer une même gamme de longueurs d'onde, notamment les UV-C et les UV-V.

Selon l'invention, les colloïdes désignent des particules sous forme cristalline. Ils peuvent également être appelés points quantiques (« quantum dots »). En milieu liquide, par exemple dans un milieu aqueux, les colloïdes forment une suspension colloïdale ou une dispersion colloïdale.

Selon un mode de réalisation préféré, les colloïdes désignent des nanostructures ou des nanocristaux.

Les colloïdes d'oxyde de bismuth $Bi_2O_3$ peuvent être synthétisés selon les techniques conventionnelles, par exemple par l'approche dite « bottom up » de croissance de précurseurs. Cette voie de synthèse, couramment utilisée dans le domaine des nanomatériaux, met en œuvre une étape de nucléation et une étape de croissance à partir d'atomes isolés. Elle permet de contrôler la taille des colloïdes.

Les colloïdes d'oxyde de bismuth $Bi_2O_3$ peuvent être synthétisés à partir de précurseurs conventionnels tels que des oxalates de bismuth, à savoir $Bi_2(C_2O_4)_3$ ou $Bi(C_2O_4)OH$, ou le nitrate de bismuth, $Bi(NO_3)_3$.

Selon un mode de réalisation particulier, la synthèse des colloïdes $Bi_2O_3$ peut être réalisée en milieu basique, par exemple en présence de soude.

Selon un autre mode de réalisation particulier, la synthèse des colloïdes $Bi_2O_3$ peut également être réalisée en présence de composés tels que l'acide nitrique et/ou la polyvinylpyrrolidone et/ou la glycérine.

Avantageusement, la synthèse des colloïdes $Bi_2O_3$ selon l'invention peut être réalisée dans l'eau.

Les cristaux de Bi203 ainsi obtenus peuvent être traités par :

- précipitation ; et/ou
- lavage, notamment par filtration ; et/ou
- calcination.

Comme déjà indiqué, le Demandeur a remarqué que les colloïdes d'oxyde de bismuth peuvent agir en tant qu'agent bloquant du rayonnement électromagnétique, avantageusement de 200 nm à 420 nm, de préférence le rayonnement ultraviolet, notamment lorsqu'ils sont appliqués sur la peau.

Selon leurs caractéristiques de symétrie morphologique et de leurs propriétés physiques, les cristaux peuvent être classés en systèmes cristallins.

L'oxyde de bismuth $Bi_2O_3$ peut présenter différentes phases cristallographiques (polymorphes) ayant des propriétés thermiques, conductrices et optiques différentes, par exemple :

- la phase $\alpha$ ; et
- la phase $\beta$ tétragonale ;
- la phase y de structure cubique centré ;
- la phase $\delta$ de structure cubique à faces centrées.

La phase $\alpha$ cristallise dans un réseau de type monoclinique dont les paramètres de maille sont : a = 5,84 Å ; b = 8,15 Å ; c = 7,50 Å ; $\beta$ = 112.97° ; Z = 4 dans le groupe d'espace $P2_{1/c}$. Cette phase présente des lacunes ordonnées avec un quart des sites oxygène libres.

En fonction des conditions de synthèse ou de température, il est possible de favoriser l'une ou l'autre de ces phases.

De manière inattendue, les colloïdes d'oxyde de bismuth dopés, avantageusement de forme monoclinique, encore plus avantageusement de forme monoclinique de phase alpha, présentent un effet bloquant sur quasiment toute la gamme des ultraviolets, c'est-à-dire à des longueurs d'onde comprises entre 200 et 420 nm.

Selon un mode de réalisation particulier, la composition selon l'invention comprend des colloïdes d'oxyde de bismuth $Bi_2O_3$ présents uniquement sous forme cristalline monoclinique.

Selon un mode de réalisation particulier, la composition selon l'invention comprend des colloïdes d'oxyde de bismuth $Bi_2O_3$ présents uniquement sous forme cristalline monoclinique de phase $\alpha$.

En d'autres termes, la composition selon l'invention est dépourvue en colloïdes d'oxyde de bismuth $Bi_2O_3$ :

- de phase $\beta$, y, et $\delta$ ; et/ou
- de forme tétragonale, cubique centré et cubique à faces centrées

Selon une caractéristique essentielle de l'invention, les colloïdes d'oxyde de bismuth sont dopés avec un agent dopant, tel qu'un métal.

Au sens de l'invention, par « dopage », on désigne la substitution d'un atome de bismuth par un atome de métal, dans la maille cristalline.

Selon l'invention, le dopage consiste à réaliser la synthèse des colloïdes d'oxyde de bismuth $Bi_2O_3$, notamment par « bottom up », et en présence d'un précurseur de l'agent dopant ou de l'agent dopant.

Le dopage au sens de l'invention est différent du dopage conventionnel qui consiste tout d'abord à synthétiser les colloïdes d'oxyde puis à les mélanger ultérieurement avec le précurseur de l'agent dopant.

Selon l'invention, le dopage ne modifie pas la configuration de la forme cristalline. En d'autres termes, le diagramme de diffraction des rayons X sur poudre des colloïdes $\alpha$-$Bi_2O_3$ (phase alpha et réseau monoclinique) se superpose exactement avec celui des colloïdes $\alpha$-$Bi_2O_3$ (phase alpha et réseau monoclinique) dopés avec un métal selon l'invention.

Au contraire, dans l'art antérieur, le dopage avec un élément, notamment un métal, des colloïdes $\alpha$-$Bi_2O_3$ modifie la forme cristalline. Il s'ensuit que les colloïdes ne sont pas uniquement sous la forme de phase alpha mais sous la forme d'un mélange des phases alpha, gamma et/ou delta.

Selon un mode de réalisation particulier, le métal est choisi dans le groupe comprenant les métaux alcalins, les métaux alcalino-terreux, les lanthanides, les actinides, les métaux de transition, les métaux pauvres, les métalloïdes et leurs assemblages. De manière avantageuse, il s'agit d'un métal de transition.

A titre d'exemple, le dopage des colloïdes d'oxyde de bismuth peut être mis en œuvre avec le fer, le manganèse, le magnésium, le cuivre, le chrome, le nickel ou encore le zinc, éventuellement à l'exception du potassium ou du calcium.

Avantageusement, le métal est le fer.

De manière générale, ce dopant représente 0,01 à 5% en masse, par rapport à la masse de $Bi_2O_3$ (avant greffage avec le polymère biocompatible), plus avantageusement 0,01 à 0,15%.

Le dopage des colloïdes d'oxyde de bismuth avec un métal, avantageusement le fer, permet d'inhiber l'activité photocatalytique des colloïdes. L'inhibition de cette activité réside dans un écartement des mailles cristallines qui empêche alors le transfert d'électron.

Outre la fonction de bloqueur du rayonnement électromagnétique, les colloïdes d'oxyde de bismuth dopés peuvent également apporter des propriétés antioxydantes, antibactériennes, bactéricides, antibiofilm, fongicides et antivirales à la composition cosmétique selon l'invention.

La cristallographie aux rayons X, également appelée radiocristallographie ou diffractométrie de rayons X, permet d'étudier la structure d'un matériau cristallin à l'échelle atomique. Cette technique s'appuie sur le phénomène physique de diffraction des rayons X. A titre d'exemple, un diffractomètre ayant une source au cuivre peut être utilisé.

Le diagramme de diffraction forme ainsi une véritable signature de la forme cristalline d'un composé. Cette signature est spécifique à la forme cristalline du composé. Elle se présente sous la forme d'une liste de pics de position en angle $2\theta$ (2-thêta).

Selon un mode de réalisation préféré de l'invention, les colloïdes d'oxyde de bismuth dopés sont avantageusement sous forme monoclinique, encore plus avantageusement sous forme monoclinique de phase alpha, alpha -$Bi_2O_3$ ou -$\alpha$-$Bi_2O_3$.

Dans un mode de réalisation particulier, les colloïdes de $Bi_2O_3$ dopés sont greffés avec un polymère biocompatible.

Les colloïdes d'oxyde de bismuth mis en œuvre dans l'invention ne sont pas comparables aux particules de l'art antérieur, par exemple celles divulguées dans les documents JP 2010-090001 et JP 2010-090002. Ces documents divulguent des particules non dopées et/ou non greffées, dont la phase cristalline ($\alpha$, $\beta$, y...) n'est accessoirement pas précisée.

Au sens de l'invention, par « biocompatible », on désigne un composé cytocompatible avec la peau, les muqueuses et les phanères et qui présente une cytotoxicité inférieure à 15%, de préférence inférieure à 10%, à l'égard d'épidermes humains reconstruits *in vitro* (modèle SkinEthic RHE). En d'autres termes, ce composé demeure quasiment neutre vis-à-vis de la viabilité cellulaire.

Cette cytocompatibilité peut être évaluée par le test de viabilité cellulaire dont le réactif est le sel de tétrazolium MTT (bromure de 3-(4,5-dimethylthiazol-2-yl)-2,5-diphenyl tetrazolium).

Le test MTT est une méthode colorimétrique de détection de l'activité mitochondriale qui permet d'évaluer le pouvoir cytotoxique d'un constituant. Il est basé sur la réduction de l'anneau de tétrazolium contenu dans le réactif, par la succinate déshydrogénase mitochondriale des cellules vivantes actives, en formazan. Ceci forme un précipité dans la mitochondrie de couleur violette.

En pratique, après application de l'élément d'essai sur des épidermes pendant 42 minutes, puis une incubation post-traitement de 42 heures, la viabilité cellulaire est évaluée par la mesure de l'activité de la succinate deshydrogénase mitochondriale des cellules vivantes. Cette enzyme transforme le MTT en cristaux de formazan bleu. Après dissolution de ces cristaux, on réalise une lecture spectrophotométrique de la densité optique à 550 nm. Les mesures d'absorbance sont proportionnelles au nombre de cellules vivantes.

Le polymère biocompatible peut avoir des propriétés hydrophiles ou lipophiles. De manière avantageuse, il s'agit d'un polymère hydrophile.

Ce polymère biocompatible peut être avantageusement hydrophile et choisi dans le groupe comprenant la polyvinylpyrrolidone (PVP) et ses copolymères tels que le triacontanyl-PVP, le PVP-eicosène ou le PVP-vinylacétate, l'acétate de polyvinyle, l'alcool polyvinylique, le polychlorure de vinyle, les styréniques, les polyamides, les acrylates, les polyesters, les polybutènes, les polysaccharides tels que le pullulane, les arabinoxylanes, la cellulose, la chitine, le chitosan, la gomme xanthane, le dextran, la gomme welan, la gomme gellane, la gomme arabique, l'acide hyaluronique, la cellulose et ses dérivées, l'amidon, le diutane, les protéines et leur constituants tels que la séricine et les acides aminés, les acides gras, les phospholipides, les phosphoglycérides, les triglycérides, les agents de couplage silanés et leurs mélanges.

Le greffage permet d'éviter la pénétration des colloïdes de $Bi_2O_3$ dans la peau grâce à l'amélioration de la bioadhésivité entre les colloïdes et la peau. En d'autres termes, l'amélioration de l'accroche des colloïdes sur la peau limite ou empêche leur pénétration dans la peau.

Grâce à des interactions physiques ou mécaniques et des interactions chimiques, le polymère biocompatible agit en tant que bioadhésif sur la peau. Ainsi, la composition selon l'invention adhère à la peau et n'est pas éliminée par simple lavage à l'eau ou à l'eau de mer.

La bioadhésion du polymère biocompatible, par création de liaisons chimiques avec la peau, entraine successivement :

- un contact intime entre le polymère biocompatible et la peau. Ce contact intime est favorisé par le mouillage du polymère biocompatible bioadhésif sur la peau et/ou son le gonflement, et
- le comblement par le polymère biocompatible bioadhésif des crevasses et ridules de la peau.

Le polymère biocompatible permet, en outre, d'améliorer la dispersion des colloïdes de $Bi_2O_3$ en milieu aqueux, et donc d'obtenir une répartition homogène lorsque la composition selon l'invention est appliquée sur la peau. La composition comprenant les colloïdes selon l'invention est donc avantageusement une composition dans laquelle les colloïdes sont en suspension.

Le greffage avec un polymère biocompatible permet également d'améliorer la stabilité des colloïdes de $Bi_2O_3$ dans le temps et/ou à pH acide, notamment au pH de la peau entre 5,2 et 7. En l'absence de greffage, les colloïdes de $Bi_2O_3$, notamment alpha-$Bi_2O_3$, sont instables dans le temps et/ou se détériorent plus rapidement à pH inférieur à 7.

La notion de greffage, ou de fonctionnalisation, des colloïdes fait partie des connaissances générales de l'homme du métier. Le greffage, ou fonctionnalisation, correspond à la formation de liaisons covalentes, par exemple, entre le polymère biocompatible et la surface des colloïdes. Il ne s'agit pas de colloïdes de type cœur/coquille.

Le polymère biocompatible peut apporter des propriétés lipophiles ou hydrophiles aux colloïdes.

De manière générale, la composition peut comprendre entre 1 et 60% de colloïdes de $Bi_2O_3$ dopés, et avantageusement greffés, en masse, par rapport à la masse de la composition, plus avantageusement entre 20 et 50%. Il s'agit du pourcentage massique des colloïdes de $Bi_2O_3$ dopés, et avantageusement greffés.

De manière avantageuse, les colloïdes de $Bi_2O_3$ dopés de la composition peuvent comprendre entre 60 et 100% de colloïdes d'oxyde de bismuth de forme cristalline alpha dopés, de préférence de forme cristalline alpha monoclinique dopés, avantageusement entre 80 et 100%, de préférence 100%. Ces colloïdes dopés sous forme cristalline alpha sont avantageusement greffés avec un polymère biocompatible. En d'autres termes, selon ce mode de réalisation, 60 à 100% des colloïdes de $Bi_2O_3$ dopés de la composition sont sous la forme cristalline alpha, avantageusement 80 à 100%, plus avantageusement 100%.

Les colloïdes de $Bi_2O_3$ sont avantageusement de forme sphérique.

Les colloïdes de $Bi_2O_3$, avantageusement la phase cristalline alpha, de préférence la phase cristalline alpha monoclinique, dopés et greffés ou non, présentent une taille avantageusement comprise entre 0,5 et 1000 nm, plus avantageusement entre 0,5 et 100 nm, et encore plus avantageusement de l'ordre de 30 nm.

La taille étant mesurée par DRX (diffraction des rayons X) qui est une technique permettant de mesurer la taille de cristaux à l'état solide.

Par « taille », on désigne la dimension la plus importante des colloïdes, par exemple le diamètre dans le cas de colloïdes de forme sphérique. Il s'agit de la taille moyenne de colloïdes greffés ou non. En effet, la taille des colloïdes greffés, selon la présente invention, est également comprise dans les plages de valeurs données ci-dessus.

Selon un mode de réalisation particulier, la composition peut comprendre en outre, un filtre solaire minéral lipophile et/ou hydrophile. Ce filtre peut notamment être choisi dans le groupe comprenant des oxydes de titane ($TiO_2$), de zinc (ZnO), de fer ($Fe_2O_3$), de zirconium ($ZrO_2$), de silicium ($SiO_2$), de manganèse (par exemple MnO), d'aluminium ($Al_2O_3$), de cérium ($Ce_2O_3$), et leurs mélanges.

Avantageusement, le filtre solaire minéral est sous forme colloïdale ou de particules.

Selon un autre mode de réalisation particulier, la composition peut comprendre en outre un filtre solaire organique lipophile et/ou hydrophile. Ce filtre peut notamment être choisi dans le groupe comprenant les désignations INCI: Camphor benzalkonium Methosulfate, Homosalate, Butyl Methoxydibenzoylmethane, Phenylbenzimidazole - Sulfonic Acid, Terephthalylidene Dicamphor Sulfonic Acid, Butyl Methoxydibenzoylmethane, Benzylidene Camphor Sulfonic Acid, Octocrylène, Polyacrylamidomethyl Benzylidène Camphor, Ethylhexyl Methoxycinnamate, PEG 25-PABA, Isomamyl p-Methoxycinnamate, Ethylhexyl Triazone, Drometrizole Trisiloxane, Diethylhexyl Butamido Triazone, 4-Methylbenzylidene Camphor, 3-Benzylidène Camphor, Ethylhexyl Salicylate, Ethylhexyl Dimethyl PABA ou Octyl Dimethyl PABA, Benzophénone-4 / Benzophénone-5, Methylène Bis- Benzotriazolyl Tetra- methylbutylphenol, Disodium Phenyl Dibenzimidazole Tetrasulfonate, Bis-Ethylhexyloxyphenol Methoxyphenyl Triazine, Polysilicone-15, Diethylamino Hydroxybenzoyl Hexyl Benzoate et leurs mélanges.

Selon un mode de réalisation particulier, la composition topique est à usage thérapeutique.

Selon un autre mode de réalisation particulier, la composition topique est à usage non-thérapeutique.

Avantageusement, la composition selon l'invention est une composition solaire.

Elle peut se présenter sous la forme d'une suspension aqueuse de colloïdes dopés de préférence avec un métal, et avantageusement greffés, d'une suspension de colloïdes dopés de préférence avec un métal, et avantageusement greffés, dans une phase huile ou d'une émulsion de type eau dans huile ou huile dans eau comprenant des colloïdes dopés de préférence avec un métal, et avantageusement greffés.

Le polymère biocompatible peut avoir des propriétés hydrophiles ou lipophiles.

Lorsque la composition comprend un dispersant de type huile, le polymère biocompatible greffé sur les colloïdes d'oxyde de bismuth est avantageusement lipophile.

Lorsque la composition comprend un dispersant de type eau, le polymère biocompatible greffé sur les colloïdes d'oxyde de bismuth est avantageusement hydrophile.

La composition selon l'invention comprend avantageusement entre 2 et 20% de polymère biocompatible en masse, par rapport à la masse de la composition, plus avantageusement entre 4 et 10%. Ces pourcentages incluent le polymère biocompatible éventuellement greffé sur les colloïdes de $Bi_2O_3$ dopés, de préférence avec un métal.

Selon un mode de réalisation particulier, la composition selon l'invention peut comprendre en outre, au moins un additif choisi dans le groupe comprenant les dispersants, les humectants, les stabilisants, et les régulateurs de pH.

Avantageusement, le dispersant est l'eau et/ou l'huile.

De préférence, l'huile est choisie dans le groupe comprenant les huiles hydrocarbonées d'origine végétale (huile de tournesol, de maïs, de soja, de pépins de raisin, de sésame, de noisette, de ricin...), les hydrocarbures (linéaires ou ramifiés) d'origine minérale ou synthétique (huile de paraffine...), les huiles de silicone (polyméthylsiloxanes, cyclopolydiméthylsiloxanes...), les huiles fluorées, et leurs mélanges.

Selon un autre mode de réalisation, l'huile peut être une huile hydrocarbonée d'origine animale.

Le dispersant est avantageusement l'eau.

En pratique, la composition selon l'invention comprend avantageusement entre 20 et 80% d'eau et/ou d'huile en masse, par rapport à la masse de la composition, plus avantageusement entre 40 et 60%.

De manière avantageuse, l'agent humectant est choisi dans le groupe comprenant le glycérol, l'urée, l'acide lactique et leurs mélanges.

La composition selon l'invention comprend avantageusement entre 5 et 25% d'agent humectant en masse, par rapport à la masse de la composition, avantageusement entre 7 et 15%.

L'agent humectant permet d'éviter que la composition ne se dessèche trop rapidement une fois qu'elle a été appliquée sur la peau. Il contribue également à l'hydratation de la peau. Il peut en outre contribuer au contrôle de la viscosité de la composition selon l'invention, et ce, dans le but d'optimiser son étalement sur la peau.

De manière avantageuse, le stabilisant est choisi dans le groupe comprenant le monolaurate de sorbitane ; la gomme guar, la gomme xanthane, et leurs mélanges.

La composition selon l'invention comprend avantageusement entre 0,5 et 5% de stabilisant en masse, par rapport à la masse de la composition, plus avantageusement entre 1 et 3%.

Le stabilisant permet de contrôler la viscosité de la composition.

De manière avantageuse, le régulateur de pH est choisi dans le groupe comprenant l'acide citrique, l'acide acétique, l'acide adipique, l'acide ascorbique, l'acide borique, l'acide fumarique, l'acide glycolique, l'acide lactique, l'acide malique, l'acide urique et leurs mélanges.

La composition selon l'invention comprend avantageusement entre 0,1 et 1% de régulateur de pH en masse, par rapport à la masse de la composition, plus avantageusement entre 0,2 et 0,5%.

Le régulateur de pH permet de maintenir le pH de la composition à une valeur physiologique. Il permet d'améliorer la stabilité de la composition en ajustant son pH lorsqu'elle est appliquée sur la peau, c'est-à-dire lorsqu'elle est appliquée sur un milieu acide.

L'homme du métier saura adapter la quantité de régulateur de pH de manière à ce que la composition présente un pH avantageusement compris entre 5,2 et 7.

Selon un mode de réalisation particulier, la composition selon l'invention est avantageusement dépourvue de conservateur, ce qui n'est pas le cas de la majorité des compositions conventionnelles de protection solaire.

Comme déjà mentionné, la composition cosmétique est une composition topique. Elle présente de nombreux avantages, parmi lesquels :

- une formulation simple contenant peu de composants, avantageusement en milieu aqueux,
- une efficacité accrue dans la protection contre un spectre large du rayonnement ultraviolet (UV-C, UV-B, UV-A et UV-V), la composition pouvant être assimilée à une peinture solaire,
- un très haut indice de protection solaire,
- peu ou pas de pénétration dans le *stratum corneum* (couche cellulaire la plus externe de la peau), protégeant ainsi les peaux sensibles et atopiques,
- une bonne adhésion à la peau, permettant une protection solaire de longue durée.

Selon un mode de réalisation préféré, la composition selon l'invention comprend, en masse par rapport à la masse de la composition :

- entre 1 et 60% de colloïdes d'oxyde de bismuth dopés, avantageusement sous la forme alpha monoclinique, α-Bi2O3, et avantageusement greffés avec un polymère biocompatible ;
- entre 2 et 20% de polymère biocompatible structurant, de préférence la polyvinylpyrrolidone (PVP) ;
- entre 5 et 25% d'agent humectant, de préférence le glycérol ;
- entre 0,5 et 5% de stabilisant, de préférence le monolaurate de sorbitane ;
- entre 0,1 et 1% de régulateur de pH, de préférence l'acide citrique ; et
- entre 20 et 80% d'eau et/ou d'huile.

Selon un mode de réalisation préféré, la composition selon l'invention comprend, en masse par rapport à la masse de la composition :

- entre 1 et 60% de colloïdes d'oxyde de bismuth dopés, avantageusement présent uniquement sous la forme alpha monoclinique, $\alpha$-Bi$_2$O$_3$, et avantageusement greffés avec un polymère biocompatible ;
- entre 2 et 20% de polymère biocompatible structurant, de préférence la polyvinylpyrrolidone (PVP) ;
- entre 5 et 25% d'agent humectant, de préférence le glycérol ;
- entre 0,5 et 5% de stabilisant, de préférence le monolaurate de sorbitane ;
- entre 0,1 et 1% de régulateur de pH, de préférence l'acide citrique ; et
- entre 20 et 80% d'eau et/ou d'huile.

De manière avantageuse, la composition selon l'invention peut être stockée dans un milieu dépourvu de dioxyde de carbone, et avantageusement dépourvu d'oxygène. Ces conditions de stockage permettent de prolonger la durée d'utilisation de la composition selon l'invention.

La présente invention concerne également l'utilisation de colloïdes d'oxyde de bismuth Bi$_2$O$_3$ dopés et avantageusement greffés avec un polymère biocompatible, en tant que filtre ultraviolet, avantageusement des UV-A, UV-B, UV-C et UV-V, de préférence pour le spectre de longueur d'onde de 200 à 420 nm. Cette utilisation est particulièrement adaptée lorsque les colloïdes sont contenus dans une composition cosmétique topique.

L'invention et les avantages qui en découlent ressortiront mieux des figures et exemples suivants donnés afin d'illustrer l'invention et non de manière limitative.

La figure 1 représente le spectre d'absorption de filtres solaires organiques, minéraux et selon un mode de réalisation préféré de l'invention ($\alpha$-Bi$_2$O$_3$ dopé).

La figure 2 représente la réflectance pour des solutions colloïdales de TiO$_2$, ZnO et $\alpha$-Bi$_2$O$_3$ dopé en fonction de la longueur d'onde.

La figure 3 représente le facteur de protection solaire SPF de compositions contenant des particules greffées de TiO$_2$, ZnO et $\alpha$-Bi$_2$O$_3$ dopé en fonction de leur pourcentage massique.

La figure 4 représente l'analyse de l'activité photocatalytique d'un filtre solaire minéral, selon un mode de réalisation préféré de l'invention ($\alpha$-Bi$_2$O$_3$ dopé) et d'un témoin.

La figure 5 représente un diagramme de diffraction des rayons X sur poudre comparant les pics des colloïdes $\alpha$-Bi$_2$O$_3$ (phase cristalline alpha monoclinique) selon l'invention et des colloïdes $\alpha$-Bi$_2$O$_3$ commerciaux.

## 1/ Synthèse des colloïdes d'oxyde de bismuth dopé

Un précurseur d'oxyde de bismuth, tel que le nitrate de bismuth pentahydraté et un précurseur de fer, tel que le chlorure de fer, sont mélangés avec de l'eau, de l'acide nitrique et de l'hydroxyde de sodium puis scellés dans un autoclave.

Le mélange réactionnel est ensuite précipité puis lavé par filtration avant de subir une étape de calcination à une température comprise entre 100 et 500°C.

Une fois que le mélange réactionnel atteint la température de consigne, la température est maintenue jusqu'à la cristallisation de l'oxyde de bismuth dopé.

Des nanocristaux d'oxyde de bismuth sphériques de 7 nm de diamètre sont récoltés.

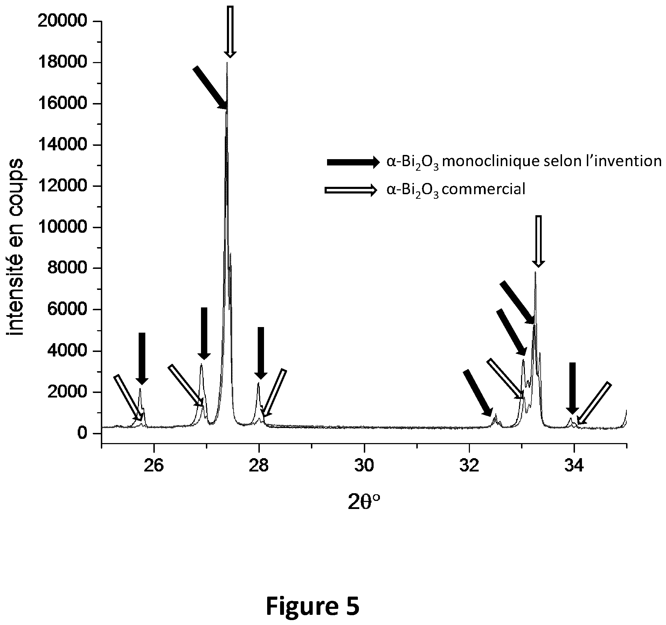

Le diagramme de diffraction des rayons X sur poudre comparant les pics des colloïdes $Bi_2O_3$ présents uniquement sous forme cristalline de phase alpha dans un réseau monoclinique selon l'invention ($\alpha$-$Bi_2O_3$) et des colloïdes $\alpha$-$Bi_2O_3$ commerciaux est représenté par la figure 5.

Les résultats montrent la présence de pics supplémentaires et/ou différents pour les colloïdes $\alpha$-$Bi_2O_3$ commerciaux par rapport aux colloïdes selon l'invention. Les colloïdes $\alpha$-$Bi_2O_3$ commerciaux correspondent à un mélange des phases alpha et gamma de l'oxyde de bismuth. En effet, le pic vers 30° 2θ n'est pas compatible avec une phase alpha mais avec une phase gamma, et les pics mineurs autour du pic principal vers 27.5° 2θ ne sont pas attendus dans une phase gamma mais se retrouvent dans une phase alpha.

En revanche, les colloïdes selon l'invention sont purs et ne présentent pas de pics supplémentaires pouvant être attribués à une autre phase cristalline de l'oxyde de bismuth que la phase alpha. En d'autres termes, les colloïdes selon l'invention correspondent uniquement à la forme cristalline en phase alpha dans un réseau monoclinique.

## 2/ Colloïdes $\alpha$-$Bi_2O_3$, $TiO_2$ et ZnO

Les spectres d'absorption de colloïdes $\alpha$-$Bi_2O_3$ monoclinique, de particules de $TiO_2$ et de particules de ZnO ont été comparés.

Pour cela, les composés suivants ont été utilisés dans de l'eau :

(a) colloïdes de $TiO_2$ (40 nm avec des agrégats mesurés par DLS de l'ordre de 250 nm) sous forme d'anatase, le $TiO_2$ étant greffé avec 2%m de PVP,
(b) colloïdes de ZnO (15 nm) greffé avec 2%m de PVP,
(c) colloïdes d'$\alpha$-$Bi_2O_3$ dopés avec du fer (colloïdes sous forme de phase alpha et dans un réseau monoclinique ; 30 nm par DRX et 40 nm par DLS) greffé 2%m de PVP.

%m désigne le pourcentage massique de PVP par rapport à la masse de $TiO_2$, ZnO ou $\alpha$-$Bi_2O_3$ dopé avec du fer.

Le greffage des colloïdes est réalisé de manière conventionnelle, par exemple dans une solution d'eau et d'éthanol contenant les colloïdes et le polymère à greffer (PVP).

La longueur d'onde produite par le diffractomètre utilisé pour mesurer la taille par DRX correspond à la raie Cu-$K_\alpha$ égale à 1,54 Å. Les autres paramètres utilisés sont les suivants: tension d'accélération : 40 kV ; courant : 40 mA ; géométrie Bragg-Brentano.

Pour les mesures de taille par DLS, les conditions sont les suivantes : longueur d'onde égale à 633 nm ; détecteur à 90° ; 25°C. L'échantillon analysé est dilué (0,5g/L) dans l'huile de cocosilicone dans une cuve de 1 mm.

### 2-a) Coefficient d'extinction massique

Les coefficients d'extinction massiques moyens (ε) de ces composés en fonction de la longueur d'onde ont été obtenus à partir de mesures effectuées au moyen d'un spectrophotomètre conventionnel. Le coefficient massique moyen correspond à la moyenne de mesures réalisées dans des cuves de 10 μm, 100 μm et 0,1 cm d'épaisseur pour des solutions de 10%m (100 g/L), 0,75%m (7,5 g/L) et 0,05%m (0,5 g/L) respectivement. Le coefficient massique est obtenu à partir de la formule de Beer-Lambert :

$$\varepsilon = A/lC$$

dans laquelle A correspond à l'absorbance mesurée, l (cm) correspond au trajet optique à travers l'échantillon et C (g/L) correspond à la concentration massique de l'échantillon.

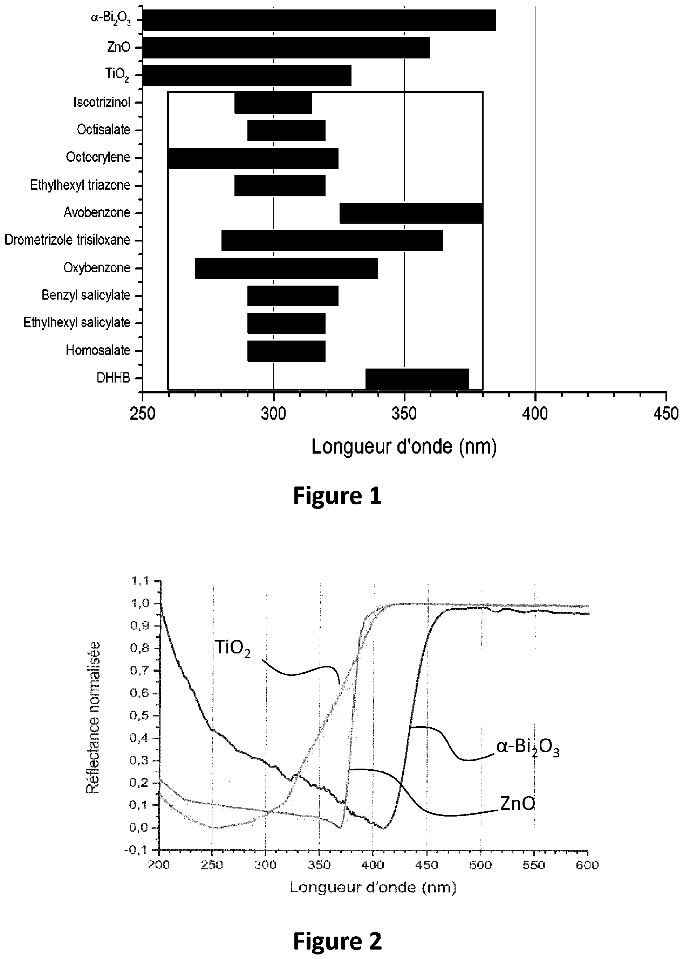

Les résultats sont représentés par la figure 2.

Les particules de $TiO_2$ présentent un coefficient massique moyen supérieur dans la région des UV-B (280-315 nm). Néanmoins, les colloïdes $\alpha$-$Bi_2O_3$ (phase alpha et réseau monoclinique) dopés présentent une capacité d'absorption des UV-A (315-400 nm) nettement supérieure à celle de $TiO_2$. Le coefficient massique moyen de ZnO reste inférieur, quelle que soit la longueur d'onde.

*2-b) Protection contre les ultraviolets*

Le facteur de protection solaire (SPF), le facteur de protection UV-A (FP-UVA) et la longueur d'onde critique des solutions (a), (b) et (c), précédemment mentionnées, ont été calculés à partir des équations suivantes, et pour une cuve de 10 $\mu$m d'épaisseur :

$$SPF = \frac{\int_{290}^{400} E_\lambda . S_\lambda . d_\lambda}{\int_{290}^{400} E_\lambda . S_\lambda . T_\lambda . d_\lambda}$$

$$SP - UVA = \frac{\int_{320}^{400} E_\lambda . S_\lambda . d_\lambda}{\int_{320}^{400} E_\lambda . S_\lambda . T_\lambda . d_\lambda}$$

$$R = \frac{\int_{290}^{\lambda} \log\frac{1}{T_\lambda} . d_\lambda}{\int_{290}^{400} \log\frac{1}{T_\lambda} . d_\lambda}$$

dans lesquelles :

$E_\lambda$ désigne le spectre d'action érythémale (valeur comprise entre 0 et 1) de la longueur d'onde $\lambda$. Il s'agit de la valeur à laquelle le rayonnement UV à la longueur d'onde $\lambda$ est susceptible de provoquer un érythème sur la peau.
$S_\lambda$ désigne l'irradiance spectrale à la longueur d'onde $\lambda$.
$T_\lambda$ désigne la transmittance de l'échantillon à la longueur d'onde $\lambda$.
$d_\lambda$ désigne une variable d'intégration.

Les valeurs $E_\lambda$ et $S_\lambda$ sont connues.
La longueur d'onde critique correspond à la longueur d'onde pour laquelle le rapport R est supérieur ou égal à 0,9.

En d'autres termes, il s'agit de la longueur d'onde pour laquelle l'intégrale de la courbe du spectre ( $\int_{290}^{\lambda} \log\frac{1}{T_\lambda} . d_\lambda$ ) est égale à 90% de l'intégrale entre 290 et 400 nm.
Les longueurs d'onde critiques des composés $TiO_2$, ZnO et $\alpha$-$Bi_2O_3$ sont respectivement égales à 368 nm, 362 nm et 382 nm.
Seul le composé $\alpha$-$Bi_2O_3$ permet à lui seul d'obtenir une longueur d'onde critique supérieure à 370 nm.

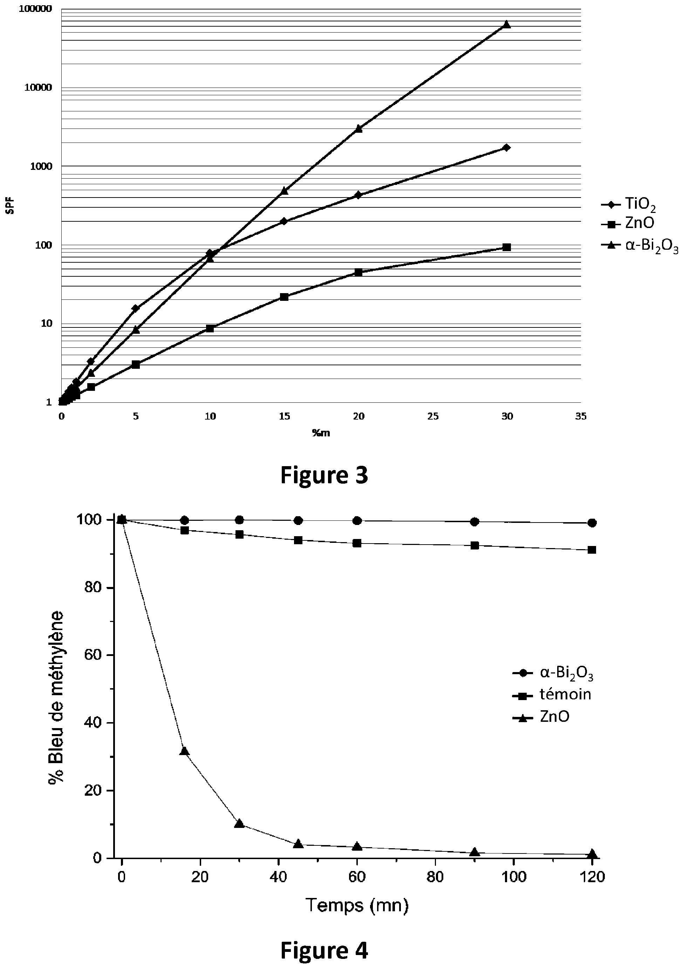

La figure 3 représente le facteur de protection solaire SPF de particules greffées de $TiO_2$, ZnO et $\alpha$-$Bi_2O_3$ (phase alpha et réseau monoclinique) dopé avec du fer en fonction de leur pourcentage massique (0,1 à 30%m) dans une cuve de 10 $\mu$m.
Ces courbes permettent de déterminer la quantité de filtre solaire nécessaire pour atteindre un facteur de protection solaire SPF prédéterminé. Le tableau 1 précise les pourcentages massiques nécessaires pour atteindre un SPF de 20, 30, 50 ou 100 à partir des données de la figure 3.

Tableau 1 : Pourcentage massique (%m) de filtre minéral ($TiO_2$, ZnO, $\alpha$-$Bi_2O_3$ dopé avec du fer) en fonction du facteur de protection solaire SPF.

|  | SPF 20 | SPF 30 | SPF 50 | SPF 100 |
|---|---|---|---|---|
| $TiO_2$ (%m) | 5,6 | 6,66 | 8,26 | 11,2 |
| ZnO (%m) | 14,5 | 17 | 21 | 31,7 |
| $\alpha$-$Bi_2O_3$ dopé (%m) | 7,06 | 8,04 | 9,3 | 11 |

Les tableaux 2 et 3 répertorient le facteur de protection UVA (SP-UVA) et le rapport SPF/SP-UVA pour des SPF de 50 et 100.

Tableau 2 : Facteur de protection UVA (SP-UVA) et rapport SPF/SP-UVA pour un SPF de 50.

|  | SP-UVA | SPF / SP-UVA |
|---|---|---|
| $TiO_2$ (8,26%m) | 5,22 | 9,57 |
| ZnO (21%m) | 8,34 | 6 |
| $\alpha$-$Bi_2O_3$ dopé (9,3%m) | 21,21 | 2,36 |

Tableau 3 : Facteur de protection UVA (SP-UVA) et rapport SPF/SP-UVA pour un SPF de 100.

|  | SP-UVA | SPF / SP-UVA |
|---|---|---|
| $TiO_2$ (11,2%m) | 8,73 | 11,46 |
| ZnO (31,7%m) | 13,27 | 7,54 |
| $\alpha$-$Bi_2O_3$ (11%m) | 36,74 | 2,72 |

Il résulte de la figure 3 que l'efficacité des filtres minéraux étudiés est la suivante : $TiO_2$ > $Bi_2O_3$ > ZnO pour des concentrations comprises entre 0,1 et 10%m ; et $Bi_2O_3$ > $TiO_2$ > ZnO pour des concentrations supérieures à 10%m.

D'autre part, les colloïdes d'$\alpha$-$Bi_2O_3$ (phase alpha et réseau monoclinique) dopés avec du fer présentent une gamme d'absorption plus importante que celle des filtres minéraux conventionnels (figure 1). Ainsi, ils permettent de ne pas avoir nécessairement recours à un mélange de filtres organiques et/ou minéraux.

3/ *Activité photocatalytique*

L'activité photocatalytique de filtres UV minéraux est évaluée par suivi de la dégradation du bleu de méthylène en présence de différents filtres ou d'un contrôle et d'un rayonnement UV.

Les filtres minéraux testés correspondent à des oxydes, Plus précisément, il s'agit de ZnO, et $\alpha$-$Bi_2O_3$ dopé avec du fer selon l'invention.

En détail, 10 $\mu$L de suspension d'oxyde à 50 g/L dans l'eau est ajoutée à 4 990 $\mu$L de solution de bleu de méthylène dans l'eau à $1E^{-5}$ mol.$L^{-1}$. Un témoin est également fait en ajoutant 10 $\mu$L d'eau à la place de la suspension d'oxyde dans 4 990 $\mu$L de solution de bleu de méthylène dans l'eau à $1E^{-5}$ mol.$L^{-1}$. Les solutions sont mise dans le noir pendant 30 minutes afin d'atteindre l'équilibre d'adsorption du colorant à la surface de l'oxyde étudié.

L'absorbance des solutions est ensuite mesurée entre 540 nm et 710 nm, ce qui correspond à l'absorption du bleu de méthylène. Ces mesures constituent le temps t=0 minutes. Les solutions sont ensuite mises sous agitation et sous illumination UV.

Des mesures d'absorbance à 15, 30, 45, 60, 90 et 120 minutes sont effectuées.

L'aire sous la courbe entre 540 nm et 710 nm permet de mesurer la quantité relative de bleu de méthylène comparée à la mesure t = 0 min.

Les résultats sont représentés par la figure 4.

Les résultats montrent que l'oxyde de zinc a une activité photocatalytique importante avec une dégradation de 90% du bleu de méthylène en 30 minutes.

Le témoin montre une dégradation d'environ 10% qui n'est pas due à de la photocatalyse, mais à une photo-dégradation du colorant sous UV.

Dans le cas des colloïdes $\alpha$-$Bi_2O_3$ (phase alpha et réseau monoclinique) dopés selon l'invention, la dégradation est quasiment nulle ce qui démontre une absence d'activité photocatalytique de l'oxyde. La photo-dégradation du colorant semble également limitée par rapport au témoin car seul 2% du bleu de méthylène ont été dégradés. Ce phénomène est dû à l'absorption d'une partie du rayonnement UV et la réduction de ROS par les colloïdes $\alpha$-$Bi_2O_3$ dopés avec du fer selon l'invention.

4/ *Composition cosmétique selon l'invention*

Une composition selon l'invention a été préparée et comparée à des compositions solaires commerciales (tableau 4).

Tableau 4 : Caractéristiques des compositions commerciales comparées à l'invention

| Composition | Filtre UV (quantité) | SPF | UV-V bloqués |
|---|---|---|---|
| Actinica Lotion | Organiques | 50+ | 3,44% |
| ISDIN Spot Prevent | Organiques | 50+ | 0% |
| Dermina | Organiques et minéraux | 50 | 19,81% |
| Bépanthène soleil crème minérale | minéraux | 50+ | 16,59% |
| Sun Bum | Organiques | 70+ | 0% |
| Invention | $\alpha$-$Bi_2O_3$ dopé avec du fer (phase alpha et réseau monoclinique) | 50+ | 25,93% |

De manière générale, le tableau 4 montre que les compositions contenant des filtres UV inorganiques sont les plus efficaces pour bloquer les UV-V (UV visible : 400-450 nm).

La composition selon l'invention présente la meilleure efficacité sur toute la gamme des UV (UV-C, UV-B, UV-A et UV-V).

Les tests de pénétration cutanée ont montré que les compositions comprenant des filtres organiques pénètrent plus profondément la peau que les compositions comprenant un mélange de filtres organiques et inorganiques ou uniquement des filtres inorganiques.

Les meilleurs résultats sont obtenus pour la composition selon l'invention. Celle-ci pénètre nettement moins la peau que les autres compositions. Comme déjà indiqué, cet effet est certainement dû à l'amélioration de la bioadhésivité des colloïdes en raison de leur greffage avec un polymère biocompatible.

## Revendications

1. Composition topique créant une barrière photonique allant du rayonnement ultraviolet au rayonnement visible comprenant des colloïdes d'oxyde de bismuth $Bi_2O_3$ sous forme cristalline, *caractérisée* **en ce que** les colloïdes d'oxyde de bismuth sont dopés avec un métal.

2. Composition selon la revendication *1, caractérisée* **en ce que** les colloïdes d'oxyde de bismuth sont sous forme monoclinique.

3. Composition selon l'une des revendications 1 ou 2, *caractérisée* en ce les colloïdes d'oxyde de bismuth sont sous forme monoclinique de phase alpha, $\alpha$-$Bi_2O_3$.

4. Composition selon l'une des revendications 1 à 3, *caractérisée* **en ce que** les colloïdes d'oxyde de bismuth sont présents uniquement sous forme monoclinique de phase alpha, $\alpha$-$Bi_2O_3$.

5. Composition selon l'une des revendications 1 à 4, *caractérisée* **en ce que** la forme monoclinique corresponde aux paramètres de maille suivants : a = 5,84 Å ; b = 8,15 Å ; c = 7,50 Å ; $\beta$ = 112.97° ; Z = 4 dans le groupe d'espace $P2_{1/c}$.

6. Composition selon la revendication 1 à 5, *caractérisée* **en ce que** le métal est le fer.

7. Composition selon la revendication 1 à 6, *caractérisée* **en ce que** les colloïdes d'oxyde de bismuth sont greffés avec un polymère biocompatible.

8. Composition selon l'une des revendications 1 à 7, **caractérisée en ce que** les colloïdes d'oxyde de bismuth sont greffés avec un polymère biocompatible choisi dans le groupe comprenant la polyvinylpyrrolidone (PVP) et ses copolymères tels que le triacontanyl-PVP, le PVP-eicosène ou le PVP-vinylacétate, l'acétate de polyvinyle, l'alcool polyvinylique, le polychlorure de vinyle, les styréniques, les polyamides, les acrylates, les polyesters, les polybutènes, les polysaccharides tels que le pullulane, les arabinoxylanes, la cellulose, la chitine, le chitosan, la gomme xanthane, le dextran, la gomme welan, la gomme gellane, la gomme arabique, l'acide hyaluronique, la cellulose et ses dérivées, l'amidon, le diutane, les protéines et leur constituants tels que la séricine et les acides aminés, les acides gras, les phospholipides, les phosphoglycérides, les triglycérides, les agents de couplage silanés et leurs mélanges..

**9.** Composition selon l'une des revendications 1 à 8, *caractérisée* **en ce qu'**elle comprend entre 1 et 60% de colloïdes d'oxyde de bismuth dopés, et avantageusement greffés avec un polymère biocompatible en masse par rapport à la masse de la composition, avantageusement entre 20 et 50%.

**10.** Composition selon l'une des revendications 1 à 9, *caractérisée* en ce que' les colloïdes de $Bi_2O_3$ dopés comprennent entre 60 et 100% de colloïdes d'oxyde de bismuth de forme cristalline alpha dopés, et avantageusement greffés avec un polymère biocompatible.

**11.** Composition selon l'une des revendications 1 à 10, *caractérisée* **en ce que** les colloïdes d'oxyde de bismuth dopés, et avantageusement greffés avec un polymère biocompatible, présentent une taille comprise entre 0,5 nm et 1000 nm, avantageusement entre 0,5 nm et 100 nm.

**12.** Composition selon l'une des revendications 1 à 11, *caractérisée* **en ce qu'**elle comprend en outre, au moins un filtre solaire minéral lipophile et/ou hydrophile choisi dans le groupe comprenant : les oxydes de titane ($TiO_2$), de zinc (ZnO), de fer ($Fe_2O_3$), de zirconium ($ZrO_2$), de silicium ($SiO_2$), de manganèse (par exemple MnO), d'aluminium ($Al_2O_3$), de cérium ($Ce_2O_3$), et leurs mélanges ; et/ou au moins un filtre solaire organique lipophile et/ou hydrophile choisi dans le groupe comprenant les désignations INCI: Camphor benzalkonium Methosulfate, Homosalate, Butyl Methoxydibenzoylmethane, Phenylbenzimidazole - Sulfonic Acid, Terephthalylidene Dicamphor Sulfonic Acid, Butyl Methoxydibenzoylmethane, Benzylidene Camphor Sulfonic Acid, Octocrylène, Polyacrylamidomethyl Benzylidène Camphor, Ethylhexyl Methoxycinnamate, PEG 25-PABA, Isomamyl p- Methoxycinnamate, Ethylhexyl Triazone, Drometrizole Trisiloxane, Diethylhexyl Butamido Triazone, 4-Methylbenzylidene Camphor, 3-Benzylidène Camphor, Ethylhexyl Salicylate, Ethylhexyl Dimethyl PABA ou Octyl Dimethyl PABA, Benzophénone-4 / Benzophénone-5, Methylène Bis- Benzotriazolyl Tetra- methylbutylphenol, Disodium Phenyl Dibenzimidazole Tetrasulfonate, Bis-Ethylhexyloxyphenol Methoxyphenyl Triazine, Polysilicone-15, Diethylamino Hydroxybenzoyl Hexyl Benzoate et leurs mélanges.

**13.** Composition selon l'une des revendications 1 à *12, caractérisée* **en ce qu'**elle comprend en outre, au moins un additif choisi dans le groupe comprenant les dispersants, les humectants, les stabilisants, et les régulateurs de pH.

**14.** Composition selon l'une des revendications précédentes, *caractérisée* **en ce qu'**il s'agit d'une composition solaire.

**15.** Composition selon l'une des revendications précédentes, *caractérisée* **en ce qu'**elle comprend, en masse par rapport à la masse de la composition :

- entre 1 et 60% de colloïdes d'oxyde de bismuth dopés sous la forme du système cristallin monoclinique de phase alpha, $\alpha$-$Bi_2O_3$, et avantageusement greffés avec un polymère biocompatible ;
- entre 2 et 20% de polymère biocompatible, de préférence la polyvinylpyrrolidone ;
- entre 5 et 25% d'humectant, de préférence le glycérol ;
- entre 0,5 et 5% de stabilisant, de préférence le monolaurate de sorbitane ;
- entre 0,1 et 1% de régulateur de pH, de préférence l'acide citrique ; et
- entre 20 et 80% d'eau et/ou d'huile.

**16.** Utilisation de colloïdes d'oxyde de bismuth, $Bi_2O_3$ dopés, et avantageusement greffés avec un polymère biocompatible, en tant que filtre ultraviolet, avantageusement pour le spectre de longueur d'onde de 200 à 420 nm.

**17.** Utilisation de colloïdes d'oxyde de bismuth, $Bi_2O_3$ dopés, avantageusement greffés avec un polymère biocompatible, en tant qu'antioxydant.

**Figure 1**

**Figure 2**

**Figure 3**

**Figure 4**

**Figure 5**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2010090001 A

- JP 2010090002 A